# EUROPEAN PATENT SPECIFICATION

(11) **EP 4 038 389 B1**
(45) Date of publication and mention of the grant of the patent: **06.12.2023**
(21) Application number: 20792724.5
(22) Date of filing: 30.09.2020
(51) Int. Cl.: G01N 33/569

(54) **METHOD FOR DIAGNOSING TUBERCULOSIS IN URINE SAMPLES**
VERFAHREN ZUR DIAGNOSE VON TUBERKULOSE IN URINPROBEN
MÉTHODE DE DIAGNOSTIC DE LA TUBERCULOSE DANS DES ÉCHANTILLONS D'URINE

(30) Priority: 30.09.2019 ZA 201906422
(43) Date of publication of application: 10.08.2022
(73) Proprietor: University of Cape Town, 7700 Cape Town (ZA)
(72) Inventor: BLACKBURN, Jonathan Michael, 7700 Cape Town (ZA); DHEDA, Keertan Unkha Jairam, 7700 Cape Town (ZA)
(74) Representative: Boult Wade Tennant LLP
(86) International application number: PCT/IB2020/059148
(87) International publication number: WO 2021/064592

(56) References cited:
- WO-A1-2013/155460
- US-A1- 2013 203 053
- KHUTSO G. PHALANE ET AL: "Differential Expression of Host Biomarkers in Saliva and Serum Samples from Individuals with Suspected Pulmonary Tuberculosis", MEDIATORS OF INFLAMMATION., vol. 2013, 1 January 2013 (2013-01-01), pages 1-10, XP055745971, GB ISSN: 0962-9351, DOI: 10.1155/2013/981984

## Description

### FIELD OF THE INVENTION

The invention relates to a method for diagnosing active tuberculosis disease (TB) in a subject, the method comprising detecting specific biomarkers including SAA1 (Serum amyloid A1) and either of RETN (Resistin) or RBP4 (Retinol binding protein) in a urine sample from the subject.

### BACKGROUND OF THE INVENTION

There is an urgent need for disruptive and accurate tests for tuberculosis (TB), which is historically the biggest killer of mankind, and now the commonest cause of death in South Africa. It is estimated that TB impacts the country's GDP by 2 to 3% per annum (about R200 billion per year).

A significant unmet need facing the development of better TB diagnostics are the lack of clinically useful biomarkers and lack of a simple and affordable sputum-independent detection platform for TB both at the bedside and in the community. The problem is particularly acute for the ∼50% of patients co-infected with HIV who fail to produce sputum or whose sputum contains very few bacteria (< 50 bacilli/ml). The current frontline PCR-based TB diagnostic test used in many TB endemic countries (Gene Xpert Ultra; Cepheid) fails to address this issue, and the existing urine lipoarabinomannan (LAM) assay (Alere Determine^{™} TB LAM Ag) is insensitive and detects less than 50% of TB-HIV co-infected patients. Compounded by the lack of effective diagnostics, almost 40% of the world's TB cases go undiagnosed or unreported. Globally this amounts to over 4 million TB cases per annum, and in South Africa, the country with the highest incidence of TB, this amounts to between -150 000 and 200 000 new TB cases per annum (WHO 2017 Global TB Report).

Phalane et al. (Mediators Inflamm 2013:981984 "Differential expression of host biomarkers in saliva and serum samples from individuals with suspected pulmonary tuberculosis") discloses the aim of searching for TB biomarkers in accessible biological samples. The study focuses on sputum and serum samples. SAA (i.e. SAA1) is identified as a serum biomarker.

### SUMMARY OF THE INVENTION

The invention is defined by the appended claims.

According to a first embodiment of the invention, there is provided a method for diagnosing tuberculosis disease (TB), the method comprising the step of testing a urine sample from a subject for the presence of SAA1 (Serum amyloid A1) and either or both of RETN (Resistin) and RBP4 (Retinol binding protein).

In particular, the urine sample may be tested for the presence of SAA1 and RETN, SAA1 and RBP4, or SAA1, RETN and RBP4.

The urine sample may also be tested for the presence of one or more additional biomarkers selected from the group consisting of LILRB4, IL18BP, SERPINA3, CD59, IGLV3.21, IGKV1.17, O53764_MYCTU (Rv0567), I6Y0W5_MYCTU (fadE19, Rv2500c), Q79FP1_MYCTU (PE_PGRS28, Rv1452c), HTPG_MYCTU (htpG, Rv2299c), RPOB_MYCTU (rpoB, Rv0667), EFTU_MYCTU (tuf, Rv0685), ACR_MYCTU (hspX, Rv2031c), CH602_MYCTU (groEL2, Rv0440), CLPP1_MYCTU (clpP1, Rv2461c) and 10kDa chaperonin (Rv3418c).

In particular, the urine sample may also be tested for the presence of IL18BP, LILRB4 or both IL18BP and LILRB4.

The urine sample may be further tested for the presence of from one to five additional biomarkers selected from the group consisting of SERPINA3, CD59, RBP4, IGLV3.21 and IGKV1.17. In particular, the urine sample may also be tested for the presence of SERPINA3, CD59 or both SERPINA3 and CD59.

The urine sample may be further tested for the presence of from one to four additional biomarkers selected from the group consisting of HtpG, PE_PGRS28, EFTU_MYCTU and 10kDa chaperonin.

The urine sample may also be tested for the presence of one or more (such as from one to 5) other biomarkers selected from the group consisting of SAA2, CRP, TSP4, A8MUE1, AXA81 and RETN. For example, the urine sample may be tested for the presence of at least SAA1, RBP4 and SAA2; for the presence of at least SAA1, RBP4, SAA2 and TSP4; for the presence of at least SAA1, RBP4 and CRP; or for the presence of SAA1, RBP4, SAA2, CRP, TSP4, A8MUE1 and RETN.

More particularly, the urine sample may be further tested for the presence of the following biomarkers:
a) SAA1, RETN and LILRB4;
b) SAA1, RETN and IL18BP;
c) SAA1, RETN, IL18BP and LILRB4;
d) SAA1, RETN, IL18BP and IGKV1.17;
e) SAA1, RETN, IL18BP, LILRB4 and SERPINA3;
f) SAA1, RETN, IL18BP, LILRB4, SERPINA3 and CD59;
g) SAA1, RETN, LILRB4, IL18BP, SERPINA3 and any two of CD59, RBP4, IGLV3.21, CADM1 and CFHR2;
h) SAA1, RBP4, HtpG and PE_PGRS28; or
i) SAA1, RBP4, HtpG, PE_PGRS28, EFTU_MYCTU and 10kDa chaperonin.

According to another embodiment of the invention, there is provided a computer implemented method for diagnosing tuberculosis disease (TB) in a subject, the computer performing steps comprising:
receiving inputted subject data comprising values for levels of SAA1 and either or both of RETN and RBP4 in a urine sample from the subject,
comparing these values with predetermined values for the biomarkers;
determining whether the subject has TB; and
displaying information regarding the diagnosis of the subject.

In certain such embodiments, the computer performs a step comprising receiving inputted subject data comprising values for levels of one or more other biomarkers in the urine sample, wherein the other biomarkers are selected from the group consisting of LILRB4, IL18BP, SERPINA3, CD59, RBP4, IGLV3.21, IGKV1.17, O53764_MYCTU (Rv0567), I6Y0W5_MYCTU (fadE19, Rv2500c), Q79FP1_MYCTU (PE_PGRS28, Rv1452c), HTPG_MYCTU (htpG, Rv2299c), RPOB_MYCTU (rpoB, Rv0667), EFTU_MYCTU (tuf, Rv0685), ACR_MYCTU (hspX, Rv2031c), CH602_MYCTU (groEL2, Rv0440), CLPP1_MYCTU (clpP1, Rv2461c), SAA2, CRP, TSP4, A8MUE1, and CH10_MYCTU (10kDa chaperonin, Rv3418c);

According to another embodiment of the invention, there is provided a computer implemented method for diagnosing tuberculosis disease (TB) in a subject, the computer performing steps comprising:
receiving inputted subject data comprising values for levels of SAA1 and RBP4 in a urine sample from the subject,
optionally receiving inputted subject data comprising values for levels of one or more other biomarkers in the sample, wherein the other biomarkers are selected from the group consisting of HtpG, PE_PGRS28, EFTU_MYCTU and 10kDa chaperonin;
comparing these values with predetermined values for the biomarkers;
determining whether the subject has TB; and
displaying information regarding the diagnosis of the subject.

### BRIEF DESCRIPTION OF THE FIGURES

Figure 1: Heatmap of human-derived proteins in urine significantly differentially expressed between TB+ and TB- individuals (FDR ≤ 0.05) using raw log2 transformed iBAQ data.
Figure 2: The human-derived proteins in active tuberculosis urine in order of most influential proteins in distinguishing TB positive from TB negative individuals, with a larger Mean Decrease in Gini Index indicating more influence.
Figure 3: Heatmap of *Mycobacterium tuberculosis*-derived proteins in urine significantly differentially expressed between TB+ and TB- individuals (FDR ≤ 0.05) using raw log2 transformed iBAQ data.
Figure 4: The *Mycobacterium tuberculosis*-derived proteins in active tuberculosis urine in order of most influential proteins in distinguishing TB positive from TB negative individuals, with a larger Mean Decrease in Gini Index indicating more influence.
Figure 5: Histogram showing area under the curve data for individual human and mycobacterial biomarkers in distinguishing TB positive from TB negative individuals.
Figure 6: Box plots showing relative abundance levels of (A) SAA1 and (b) RPB4 in TB positive (n=60) and TB negative (n=58) individuals.
Figure 7: Heatmap of human-derived proteins in urine significantly differentially expressed between TB+ and TB- individuals using iBAQ data.
Figure 8: The human-derived proteins in active tuberculosis urine in order of most influential proteins in distinguishing TB positive from TB negative individuals, with larger decreases on removal of the feature indicating greater predictive power.
Figure 9: Box plots showing distribution and frequency of biomarkers.

### DETAILED DESCRIPTION OF THE INVENTION

A method and computer-implemented method for diagnosing tuberculosis (TB) in a urine sample from a subject are described herein. Preferably, the method is for diagnosing active TB (signs and symptoms of TB and/ or consistent imaging evidence together with microbiological confirmation (culture positivity and/ or presence of amplified DNA)), rather than for diagnosing latent M. *tuberculosis* infection (LTBI) or incipient TB. The method is independent of HIV co-infection status.

The method comprises testing a urine sample from an individual suspected of having TB for the presence of at least two human-derived biomarkers, of which one is SAA1 and the other one is either RETN or RBP4. In one embodiment, the method comprises testing the urine sample for at least SAA1 and RETN. In another embodiment, the method comprises testing the urine sample for at least SAA1 and RBP4. In a further embodiment, the method comprises testing the urine sample for at least SAA1, RETN and RBP4. The method further comprises diagnosing the individual as having or not having TB.

Optionally, the sample can also be tested for the presence or absence of other human-derived biomarkers, such as LILRB4, IL18BP, SERPINA3, CD59, IGLV3.21, IGKV1.17, SAA2, CRP, TSP4, AXA81 and A8MUE1, or any combination thereof. In particular, the urine sample may also be tested for the presence of IL18BP, LILRB4 or both IL18BP and LILRB4, and/or may also be tested for the presence of SERPINA3, CD59 or both SERPINA3 and CD59, and/or may also be tested for the presence of SAA2, TSP4, CRP and/or A8MUE1.

The sample can also be tested for the presence of one or more Mycobacterium-derived biomarkers that the applicant has identified in urine, such as those listed in Table 1.

**Table 1: M. tuberculosis proteins identified in urine.**

| |
|---|
| >tr\|O53764\|O53764_MYCTU Probable methyltransferase/methylase OS=M. tuberculosis (strain ATCC 25618 / H37Rv) GN=Rv0567 PE=1 SV=3 |
| >sp\|P9WFC9\|Y1636_MYCTU Universal stress protein Rv1636 OS= M. tuberculosis (strain ATCC 25618 / H37Rv) GN=Rv1636 PE=1 SV=1 |
| >sp\|P9WG25\|TKT_MYCTU Transketolase OS= M. tuberculosis (strain ATCC 25618 / H37Rv) GN=tkt PE=1 SV=1 |
| >sp\|P9WGC7\|SUCD_MYCTU Succinate--CoA ligase [ADP-forming] subunit alpha OS= M. tuberculosis (strain ATCC 25618 / H37Rv) GN=sucD PE=1 SV=1 |
| >sp\|P9WGY7\|RPOC_MYCTU DNA-directed RNA polymerase subunit beta OS= M. tuberculosis (strain ATCC 25618 / H37Rv) GN=rpoC PE=1 SV=1 |
| >sp\|P9WGY9\|RPOB_MYCTU DNA-directed RNA polymerase subunit beta OS= M. tuberculosis (strain ATCC 25618 / H37Rv) GN=rpoB PE=1 SV=1 |
| >sp\|P9WH29\|RS7_MYCTU 30S ribosomal protein S7 OS=M. tuberculosis (strain ATCC 25618 / H37Rv) GN=rpsG PE=1 SV=1 |
| >sp\|P9WH43\|RS1_MYCTU 30S ribosomal protein S1 OS= M. tuberculosis (strain ATCC 25618 / H37Rv) GN=rpsA PE=1 SV=1 |
| >sp\|P9WH45\|RS19_MYCTU 30S ribosomal protein S19 OS= M. tuberculosis (strain ATCC 25618 / H37Rv) GN=rpsS PE=1 SV=1 |
| >sp\|P9WHN5\|PUP_MYCTU Prokaryotic ubiquitin-like protein Pup OS= M. tuberculosis (strain ATCC 25618 / H37Rv) GN=pup PE=1 SV=1 |
| >sp\|P9WMJ7\|HTPG_MYCTU Chaperone protein HtpG OS= M. tuberculosis (strain ATCC 25618 / H37Rv) GN=htpG PE=1 SV=1 |
| >sp\|P9WMJ9\|DNAK_MYCTU Chaperone protein DnaK OS= M. tuberculosis (strain ATCC 25618 / H37Rv) GN=dnaK PE=1 SV=1 |
| >sp\|P9WMK1\|ACR_MYCTU Alpha-crystallin OS= M. tuberculosis (strain ATCC 25618 / H37Rv) GN=hspX PE=1 SV=1 |
| >sp\|P9WMV7\|Y492_MYCTU Uncharacterized GMC-type oxidoreductase Rv0492c OS= M. tuberculosis (strain ATCC 25618 / H37Rv) GN=Rv0492c PE=1 SV=1 |
| >sp\|P9WN83\|G3P_MYCTU Glyceraldehyde-3-phosphate dehydrogenase OS= M. tuberculosis (strain ATCC 25618 / H37Rv) GN=gap PE=1 SV=1 |
| >sp\|P9WNM7\|EFG_MYCTU Elongation factor G OS= M. tuberculosis (strain ATCC 25618 / H37Rv) GN=fusA PE=1 SV=1 |
| >sp\|P9WNN1\|EFTU_MYCTU Elongation factor Tu OS= M. tuberculosis (strain ATCC 25618 / H37Rv) GN=tuf PE=1 SV=1 |
| >sp\|P9WPC5\|CLPP1_MYCTU ATP-dependent Clp protease proteolytic subunit 1 OS= M. tuberculosis (strain ATCC 25618 / H37Rv) GN=clpP1 PE=1 SV=1 |
| >sp\|P9WPC9\|CLPC1_MYCTU ATP-dependent Clp protease ATP-binding subunit ClpC1 OS= M. tuberculosis (strain ATCC 25618 / H37Rv) GN=clpC1 PE=1 SV=1; |
| >sp\|P9WPD1\|CLPB_MYCTU Chaperone protein CIpB OS=Mycobacterium tuberculosis (strain ATCC 25618 / H37Rv) |
| >sp\|P9WPE5\|CH10_MYCTU 10 kDa chaperonin OS=M. tuberculosis (strain ATCC 25618 / H37Rv) GN=qroS PE=1 SV=1 |
| >sp\|P9WPE7\|CH602_MYCTU 60 kDa chaperonin 2 OS= M. tuberculosis (strain ATCC 25618 / H37Rv) GN=proEL2 PE=1 SV=1 |
| >sp\|P9WPU5\|ATPB_MYCTU ATP synthase subunit beta OS= M. tuberculosis (strain ATCC 25618 / H37Rv) GN=atpD PE=1 SV=1 |
| >sp\|P9WPU7\|ATPA_MYCTU ATP synthase subunit alpha OS= M. tuberculosis (strain ATCC 25618 / H37Rv) GN=atpA PE=1 SV=1 |
| >sp\|P9WQN5\|ARC_MYCTU Proteasome-associated ATPase OS= M. tuberculosis (strain ATCC 25618 / H37Rv) GN=mpa PE=1 SV=1 |
| >tr\|I6Y0W5\|I6Y0W5_MYCTU Acyl-CoA dehydrogenase OS=M. tuberculosis (strain ATCC 25618 / H37Rv) GN=fadE19 PE=1 SV=1 |
| >tr\|P71658\|P71658_MYCTU Putative integration host factor MihF OS= M. tuberculosis (strain ATCC 25618 / H37Rv) GN=mihF PE=1 SV=1 |
| >tr\|Q79FP1\|Q79FP1_MYCTU PE_PGRS family protein PE_PGRS28 OS= M. tuberculosis (strain ATCC 25618 / H37Rv) N=PE_PGRS28 PE=4 SV=1 |

More particularly, the sample can be tested for one or more of the following *M. tuberculosis-*derived proteins: O53764_MYCTU (Rv0567), I6Y0W5_MYCTU (fadE19, Rv2500c), Q79FP1_MYCTU (PE_PGRS28, Rv1452c), HTPG_MYCTU (htpG, Rv2299c), RPOB_MYCTU (rpoB, Rv0667), EFTU_MYCTU (tuf, Rv0685), ACR_MYCTU (hspX, Rv2031c), CH602_MYCTU (groEL2, Rv0440), CLPP1_MYCTU (clpP1, Rv2461c) and/or CH10_MYCTU (10kDa chaperonin, Rv3418c). Even more particularly, the sample can be tested for the presence of HtpG, PE_PGRS28, EFTU_MYCTU and/or 10kDa chaperonin. For example, the Mycobacterium-derived proteins that are tested can be HtpG and PE_PGRS28; HtpG, PE_PGRS28 and 10kDa chaperonin; HtpG, PE_PGRS28 and EFTU_MYCTU; PE_PGRS28, or EFTU_MYCTU and 10kDa chaperonin.

The following biomarker panels have been identified as being particularly useful:
a) SAA1 and RETN;
b) SAA1, RETN and LILRB4;
c) SAA1, RETN and IL18BP;
d) SAA1, RETN, IL18BP and LILRB4;
e) SAA1, RETN, IL18BP and IGKV1.17;
f) SAA1, RETN, IL18BP, LILRB4 and SERPINA3;
g) SAA1, RETN, IL18BP, LILRB4, SERPINA3 and CD59;
h) SAA1, RETN, LILRB4, IL18BP, SERPINA3 and any two of CD59, RBP4, IGLV3.21, CADM1 and CFHR2;
i) SAA1 and RBP4;
j) SAA1, RBP4 and SAA2;
k) SAA1, RBP4 and CRP;
l) SAA1, RBP4, SAA2, TSP4;
m) SAA1, RBP4, SAA2, TSP4 and one or more of CRP, A8MUE1, RETN;
n) SAA1, RBP4, SAA2, TSP4 and CRP;
o) SAA1, RBP4, SAA2, TSP4 and A8MUE1;
p) SAA1, RBP4, SAA2, TSP4 and RETN;
q) SAA1, RBP4, SAA2, CRP;
r) SAA1, RBP4, SAA2, CRP and AXA81;
s) SAA1, RBP4, SAA2, CRP and TSP4;
t) SAA1, RBP4, SAA2, CRP, AXA81 and TSP4;
u) SAA1, RBP4, SAA2, CRP, AXA81, TSP4 and A8MUE1;
v) SAA1, RBP4, HtpG and PE_PGRS28;
w) SAA1, RBP4, HtpG, PE_PGRS28 and EFTU_MYCTU;
x) SAA1, RBP4, HtpG, PE_PGRS28 and 10kDa chaperonin;
y) SAA1, RBP4, HtpG, PE_PGRS28, EFTU_MYCTU and 10kDa chaperonin.

The method can be used to diagnose both pulmonary TB and extrapulmonary TB, such as tuberculous pleurisy, tuberculous meningitis, urogenital tuberculosis, miliary tuberculosis and tuberculosis in the bones and joints, among others.

As used herein, the terms "comprises", "comprising", "includes", "including", "contains", "containing", and any variations thereof, are intended to cover a non-exclusive inclusion and do not exclude other elements not specifically mentioned.

The terms "marker" and "biomarker" are used interchangeably to refer to a target molecule that indicates or is a sign of a normal or abnormal process in an individual or of a disease or other condition in an individual. More specifically, a "marker" or "biomarker" is an anatomic, physiologic, biochemical, or molecular parameter associated with the presence of a specific physiological state or process, whether normal or abnormal. Biomarkers can include small molecules, peptides, proteins, and nucleic acids.

"Biomarker value", "value", "biomarker level", and "level" are used interchangeably to refer to a measurement that is made using any analytical method for detecting the biomarker in a biological sample and that indicates the presence, absence, absolute amount or concentration, relative amount or concentration, titer, a level, an expression level, a ratio of measured levels, or the like, of, for, or corresponding to the biomarker in the biological sample. The exact nature of the "value" or "level" depends on the specific design and components of the particular analytical method employed to detect the biomarker.

Biomarkers are generally described as being over-expressed, under-expressed or differentially expressed as compared to an expression level or value of the biomarker that indicates or is a sign of a normal process or an absence of a disease or other condition in an individual. Thus, "over-expression", "under-expression" and "differential expression" of a biomarker can also be referred to as a variation from a "normal" or "control" expression level of the biomarker.

In one embodiment, the number of biomarkers useful for a biomarker subset or panel is based on the sensitivity and specificity value for the particular combination of biomarker values. The terms "sensitivity" and "specificity" are used herein with respect to the ability to correctly classify the TB diagnosis for an individual, based on the biomarker values detected in their biological sample. "Sensitivity" indicates the performance of the biomarkers with respect to correctly classifying individuals that have a positive TB diagnosis, i.e. evidence of disease (EVD). "Specificity" indicates the performance of the biomarkers with respect to correctly classifying individuals who have a negative TB diagnosis (NED). For example, 85% specificity and 90% sensitivity for a panel of markers used to test a set of control samples and TB diagnosis samples indicates that 85% of the control samples were correctly classified as NED samples by the panel, and 90% of the positive samples were correctly classified as EVD samples by the panel.

As described in more detail below, a label free proteomic approach was adopted to survey the urinary proteomes of 120 individuals who had been clinically stratified into TB+/HIV-, TB+/HIV+, TB-/HIV+, and TB-/HIV- groups. The consensus urinary proteome of these 120 individuals comprised 1870 proteins, including 26 of Mycobacterial origin. The set of proteins in the urine appears to vary greatly between individuals, as might be expected.

Sets of *Mycobacterium tuberculosis*-specific proteins and human proteins that are differentially expressed in the urine of patients with active TB and which are suitable for diagnosing TB were identified. These sets encompass the gene identifiers, proteins and/or peptides of Table 2 and their downstream functions in either M. *tuberculosis* bacilli or humans. Notably, these biomarker panels were identified in the urine of both HIV negative and HIV positive individuals. To the knowledge of the applicant, the biomarker panels have not been reported in the urine of suspected TB patients. Moreover, it could not have been arrived at starting from knowledge of the individual proteins that comprise the panels or from knowledge of proteins found in other body fluids of TB patients, as many proteins are considerably degraded before they end up in urine.

**Table 2: Top candidate human and mycobacterial biomarkers identified in urine samples.**

| | **Biomarkers ID** | **Gene ID** | **Protein** |
|---|---|---|---|
| | SAA1_HUMAN | SAA1 | Serum amyloid A1 |
| | SAA2_HUMAN | SAA2 | Serum amyloid A2 |
| | Q5VY30_HUMAN | RBP4 | Retinol binding protein |
| | CRP_HUMAN | CRP | C reactive protein |
| | TSP4_HUMAN | THBS4 | Thrombospondin 4 |
| | A8MUE1 | LILRB4 | Leukocyte immunoglobulin-like receptor subfamily B member 4 |
| | RETN_HUMAN | RETN | Resistin |
| | AXA81 | ANXA8L1 | Annexin A8-like protein 1 |
| | S4R371 | FABP3 | Fatty acid-binding protein, heart |
| | KV117 | IGKV1-17 | Immunoglobulin Kappa Variable 1-17 |
| | AACT | SERPINA3 | Serpin Family A Member 3 |
| | E9PNW4 | CD59 | CD59 glycoprotein |
| | I18BP | IL18BP | Interleukin-18-binding protein |
| | A0A087X0T8 | CADM1 | Cell Adhesion Molecule 1 |
| | O53764_MYCTU | Rv0567 | Probable methyltransferase/ methylase |
| | I6YOW5_MYCTU | fadE19, Rv2500c | Acyl-CoA dehydrogenase |
| | Q79FP1_MYCTU | PE_PGRS28, Rv1452c | PE-PGRS family protein |
| | HTPG_MYCTU | htpG, Rv2299c | Chaperone protein HtpG |
| | RPOB_MYCTU | rpoB, Rv0667 | DNA-directed RNA polymerase subunit beta |
| | EFTU_MYCTU | tuf, Rv0685 | Elongation factor Tu |
| | ACR_MYCTU | hspX, Rv2031c | Alpha-crystallin |
| | CH602_MYCTU | groEL2, Rv0440 | 60 kDa chaperonin 2 |
| | CLPP1_MYCTU | clpP1, Rv2461c | ATP-dependent Clp protease proteolytic subunit 1 |
| | CH10_MYCTU | groS, Rv3418c | 10 kDa chaperonin |

From the larger collection of biomarkers of Table 2, core sets of two human-derived proteins were identified for diagnosing TB (SAA1 and RETN; SAA1 and RBP4). Optionally, other human-derived proteins, such as those listed in Table 2, can be added to these two proteins to create permutations of the biosignature.

Panels of 3, 4, 5, 6 and 7 host-derived proteins were also identified which have high sensitivity and specificity.

These proteins appear to be detected reliably in the urine of TB+ individuals but not TB-individuals. A host-derived biomarker signature in the urine may seem unreliable, given the high variability in individual urine samples, and especially given that some of the top identified biomarkers are acute phase inflammatory response proteins, but in this cohort the delineation of TB+/TB- was explained almost entirely by the presence or absence of these proteins in the urine. Taken together, the combination of this proposed set of biomarkers may contribute to their specificity for TB diagnosis as opposed to other inflammatory or non-TB respiratory diseases.

One or more mycobacterial proteins in Table 1 can also be added to create a combinatorial and multidimensional biosignature comprising both human host-specific biomarkers and M. *tuberculosis-specific* biomarkers found specifically in urine. The selection of biomarkers that are both *Mycobacterium tuberculosis* and human-derived proteins and/or peptides allows for both ruling-in (positive predictive test value (PPV) >0.8) and ruling-out (negative predictive test value (NPV) >0.8) of active tuberculosis.

In one embodiment, a positive diagnosis for TB will be made when two, three, four or more of the tested biomarkers are detected, or when the levels of the detected biomarkers are higher than a typical level of the same biomarker in subjects without TB.

Cut-off or threshold values can be determined based on levels of biomarkers which are typically found in subjects with or without active TB, and the measured levels of the biomarkers detected in the sample can be compared to the cut-off levels when making the determination of whether or not the subject has active TB. In other words, the method will detect whether the biomarkers in the panels are under- or over-expressed relative to a subject who does not have active TB.

In one embodiment, the urine sample is assayed to detect the presence of the biomarkers of interest and to determine a biomarker value for each of the biomarkers (typically measured as marker RFU (relative fluorescence units)). Once a biomarker has been detected and a biomarker value assigned, each marker is scored or classified according to various methods known in the art. The marker scores are then combined to provide a total evaluation score, which reflects whether the individual has evidence of disease.

A computer with software for comparing, scoring and/or classifying the biomarker levels can be used to make the diagnosis of whether or not the subject has TB.

In some embodiments, capture agents are used to bind specifically to each of the biomarkers, and an indicator will indicate when binding of the capture agents and each of the biomarkers occurs. The capture agent is typically an antibody, but examples of other possible capture agents include affybodies, ankyrin repeat proteins, armadillo repeat proteins, nucleic acid aptamers, peptides, carbohydrate ligands, synthetic ligands, and synthetic polymers.

The term "antibody" encompasses antibodies and antibody fragments thereof, derived from any antibody-producing mammal (e.g. mouse, rat, rabbit, and primate including human), that specifically bind to a polypeptide target of interest. Exemplary antibodies include polyclonal, monoclonal and recombinant antibodies; multispecific antibodies (e.g. bispecific antibodies); humanized antibodies; murine antibodies; chimeric, mouse-human, mouse-primate, primatehuman monoclonal antibodies; and anti-idiotype antibodies, and may be any intact molecule or fragment thereof. An antibody fragment is a portion derived from or related to a full-length antibody, preferably including the antigen binding or variable region thereof.

The indicator can be a calorimetric, electrochemical, chromogenic, optical, fluorescent or radio-labeled indicator, or the like.

In one embodiment, the urine sample can be brought in contact with a solid phase support or a carrier. "Solid support" refers herein to any substrate having a surface to which molecules may be attached, directly or indirectly, through either covalent or non-covalent bonds. A "solid support" can have a variety of physical formats, which can include, for example, a membrane; a chip (e.g. a protein chip); a slide (e.g. a glass slide or coverslip); a column; a hollow, solid, semi-solid, pore- or cavity-containing particle, such as, for example, a bead; a gel; a fiber, including a fiber optic material; a matrix; and a sample receptacle. Exemplary sample receptacles include sample wells, tubes, capillaries, vials, and any other vessel, groove or indentation capable of holding a sample. A sample receptacle can be contained on a multisample platform, such as a microtiter plate, slide, microfluidics device, and the like. A support can be composed of a natural or synthetic material, an organic or inorganic material. The composition of the solid support on which capture reagents are attached generally depends on the method of attachment (e.g. covalent attachment). Other exemplary receptacles include microdroplets and microfluidic controlled or bulk oil/aqueous emulsions within which assays and related manipulations can occur. Suitable solid supports include, for example, plastics, resins, polysaccharides, silica or silica-based materials, functionalized glass, modified silicon, carbon, metals, inorganic glasses, membranes, nylon, natural fibers (such as, for example, silk, wool and cotton), polymers, and the like. The material composing the solid support can include reactive groups such as, for example, carboxy, amino, or hydroxyl groups, which are used for attachment of the capture reagents. Polymeric solid supports can include, e.g. polystyrene, polyethylene glycol tetraphthalate, polyvinyl acetate, polyvinyl chloride, polyvinyl pyrrolidone, polyacrylonitrile, polymethyl methacrylate, polytetrafluoroethylene, butyl rubber, styrenebutadiene rubber, natural rubber, polyethylene, polypropylene, (poly)tetrafluoroethylene, (poly)vinylidenefluoride, polycarbonate, and polymethylpentene. Suitable solid support particles that can be used include, e.g. encoded particles, such as Luminex^{®}-type encoded particles, magnetic particles, and glass particles.

The biosignature may be used on a suitable immunoassay detection platform that may include optical, SERS, fluorescent, electrochemical, caloric, colorimetric or other detection platforms. In particular, antibodies that are able to capture the biomarkers of the biosignature onto a surface for multiplexed quantitation in ELISA and/or lateral flow formats can be created. These can then be used to develop point-of-care diagnostic tests for TB disease.

A kit can also be provided for performing the invention. The kit is not claimed as such.

Unlike other commercially available methods, the present method can distinguish between active and latent tuberculosis, which is important for determining treatment of the subject. Active tuberculosis is the disease state where the biological fluid or tissue is smear microscopy- or culture positive for *M*. *tuberculosis,* or where *M*. *tuberculosis* is detectable by a nucleic acid amplification test, in a subject with clinical and radiological features of active TB disease; constitutional symptoms are often present. Latent tuberculosis infection is where potentially viable *M*. *tuberculosis* is present in human tissues but the individual is asymptomatic, and without clinico-radiological features of active disease.

The invention will now be described in more detail by way of the following non-limiting examples.

### Example 1:

### Materials and Methods

### Cohort recruitment and clinical classification

Patients were recruited at TB clinics in the Western Cape, South Africa. Patients were classified according to the results of chest X-ray, culture/smear, GeneXpert, and LAM tests into four clinical groups: TB+/HIV+, TB+/HIV-, TB-/HIV+, and TB-/HIV-. First pass urine samples were collected mid-stream and stored at -20 °C until processing. Samples were blinded initially, and were processed in randomised batches of 12.

### Protein precipitation

Urine samples were thawed at room temperature and vortexed to homogenise any sedimentary material. Aliquots of 4 ml were transferred into glass vials for protein precipitation with methanol and chloroform at a ratio of 1: 0.75. The precipitate was separated into phases by centrifugation at 4000 rpm for 2 minutes, and the top phase was removed carefully. The remaining protein precipitate was washed with 1 volume of methanol, and pelleted by centrifugation at 4000 rpm for 2 minutes. The supernatant was removed and the pellet was dried in a fume hood for 1 hour. The pellet was then resuspended in 200 µl denaturation buffer (6 M urea, 2 M thiourea in 10 mM tris pH 8.0) with vortexing, and the sample was then transferred to an Eppendorf tube for storage at -20 °C until use.

### In-solution tryptic digest

Protein samples were quantified using a modified Bradford assay (Bio-Rad) with the addition of 90 µl of 0.1 M HCl. A 100 µg aliquot of each sample was transferred into a fresh Eppendorf tube. Samples were reduced with 1 mM DTT (dithiothreitol) for 1 hour at room temperature, alkylated with 5.5 mM iodoacetamide for 20 minutes at room temperature, and then diluted 5x with 50 mM ammonium bicarbonate. Trypsin (New England Biolabs) was added to each sample at a ratio of 50: 1, and the samples were incubated at room temperature overnight for 16 hours. Tryptic digest was halted with the addition of 0.1% formic acid (FA).

### C18 stage tip desalting

Tryptic peptides were desalted on in-house made C18 stage tips. The C18 plugs were first activated with solvent B (60% acetonitrile (ACN), 0.1% FA), then equilibrated with solvent A (2% ACN, 0.1% FA). For each sample, 10 µg of peptides was bound to the C18 by slow centrifugation, then washed 3x with solvent A. A glass insert was placed into the Eppendorf tube and the desalted peptides were eluted 3x with 40 µl of solvent C (80% ACN, 0.1% FA) into the glass insert. The peptides were then dried in a vacuum centrifuge at 35°C for 1 hour, and stored at -20°C until use.

### Liquid chromatography mass spectrometry (LCMS)

Peptide samples were reconstituted in solvent A to a final concentration of 200 ng/µl. Samples were loaded into the Dionex Ultimate 3000 autosampler in randomised batches. A total of 400 ng of each sample were loaded onto an in-house packed 4 cm Luna C18 5 µm trap column at a flow rate of 5 µl/min. The valve was then switched to bring the trap column in line with an in-house packed 40 cm Aeris peptide C18 3.6 µm analytical column (ID 75 µm). The mobile phase gradient went from 6-35% B (ACN with 0.1% FA) over 190 minutes at 0.4 pl/min and 40°C. Analytes eluted directly into a Thermo QExactive hybrid quadrupole Orbitrap instrument, which acquired MS and MS/MS scans in top 10 mode. MS scans were acquired at a resolution of 70000 with an AGC target of 3e6 or an injection time of 250 ms. MS/MS scans were acquired at a resolution of 17500 with an AGC target of 5e4 or an injection time of 80 ms. The dynamic exclusion window was 30 seconds and the normalised collision energy was 28.

### Data processing

Data were analysed using MaxQuant version 1.5.3.12 with match between runs, iBAQ, and LFQ on. The database used was downloaded from Uniprot in August 2016 and was a combined *Homo sapiens* and *Mycobacterium tuberculosis* H37Rv database. The MaxQuant output folder '.txt' was analysed using an in-house QC pipeline in R to assess the quality of the runs. Contaminant and reverse hits were removed from the data. Once the MaxQuant analysis was complete, the clinical stratification of each sample was revealed and the samples were unblinded prior to statistical analysis.

### Statistical analysis

Downstream analyses of MaxQuant iBAQ data were performed in R using the R packages caret (Kuhn (2008), "Building Predictive Models in R Using the caret" www.istatsoft.org/article/view/v028i05/v28i05.pdf) and randomForest (Svetnik, V., Liaw, A., Tong, C. and Wang, T., "Application of Breiman's Random Forest to Modeling Structure-Activity Relationships of Pharmaceutical Molecules", MCS 2004, Roli, F. and Windeatt, T. (Eds.) pp. 334-343). The dataset was randomly divided (irrespective of clinical groups) into 2/3 training set (N=79, 41 TB+ 38 TB-) and 1/3 testing set (N=39, 20 TB-, 19 TB+). Random Forest was performed on the training set with repeated 10-fold cross-validation. Next, the analysis was rerun on the training set, this time using only the top four most influential features (based on 10-fold cross-validation, four features would be sufficient to minimize the prediction error to ~9%). Finally, the predictive value of the random forest (RF) classifier was evaluated in the hold-out test dataset (N=39) with these top four predictive features.

Differential abundance testing was performed on log2-transformed iBAQ values (first setting zero values to 1), using the R package edgeR (Robinson MD, McCarthy DJ and Smyth GK (2010). edgeR: a Bioconductor package for differential expression analysis of digital gene expression data. Bioinformatics 26, 139-140). Raw iBAQ data were first filtered by abundance, removing proteins that were present in < 5% of samples, where FDR ≤ 0.05 and fold-change ≥ 1.5 was considered significant (Benjamini-Hochberg correction).

Additional statistical analysis was performed using Statistica version 13.3.

### Results

A total of 1870 protein groups were confidently identified with an FDR < 1% - 26 of Mycobacterial origin, and the remainder of human origin. The frequency of individual Mycobacterial peptides in each sample was low, with most individual Mycobacterial peptides identified in less than 10% of samples. However, both the human and Mycobacterial proteins were usually identified by multiple unique peptides, with 1672 proteins identified by 2 or more unique peptides.

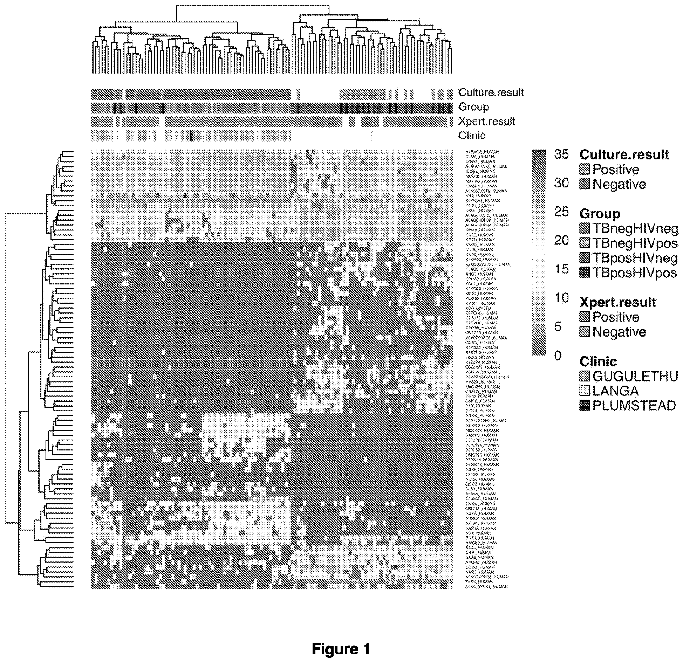

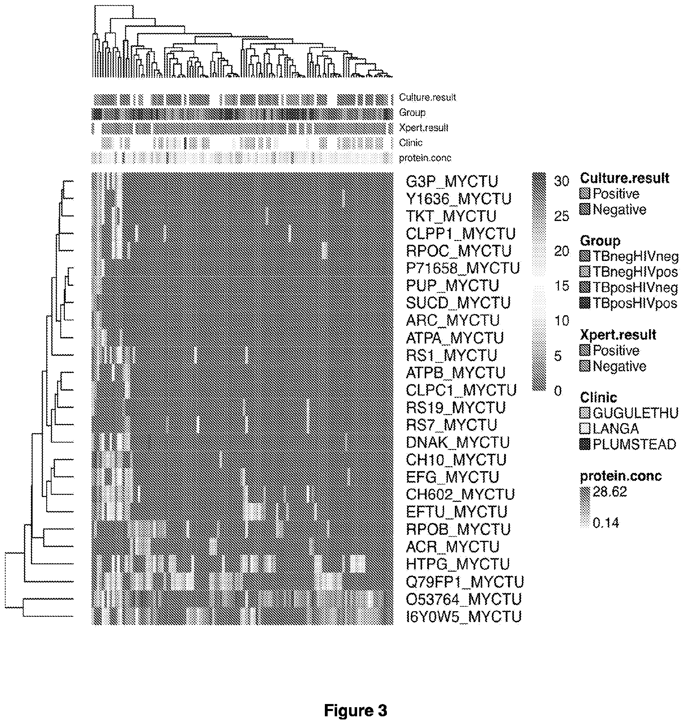

Differential abundance testing was performed on iBAQ values to identify proteins significantly differentially abundant between TB+ and TB- individuals (Figures 1 and 3). There were 102 differentially abundant proteins with an FDR ≤ 5% and fold-change ≥ 1.5. Functional enrichment analysis was carried out on these proteins using String DB to identify enriched GO terms and pathways.

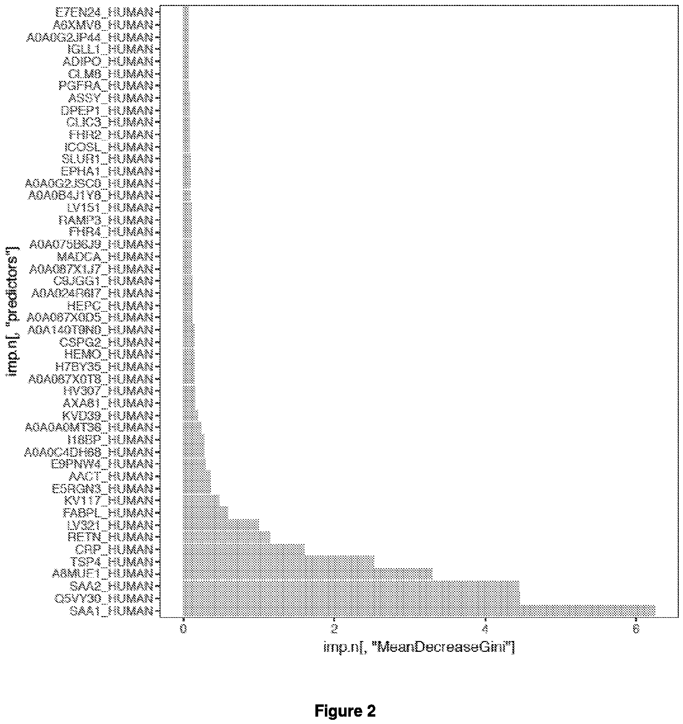

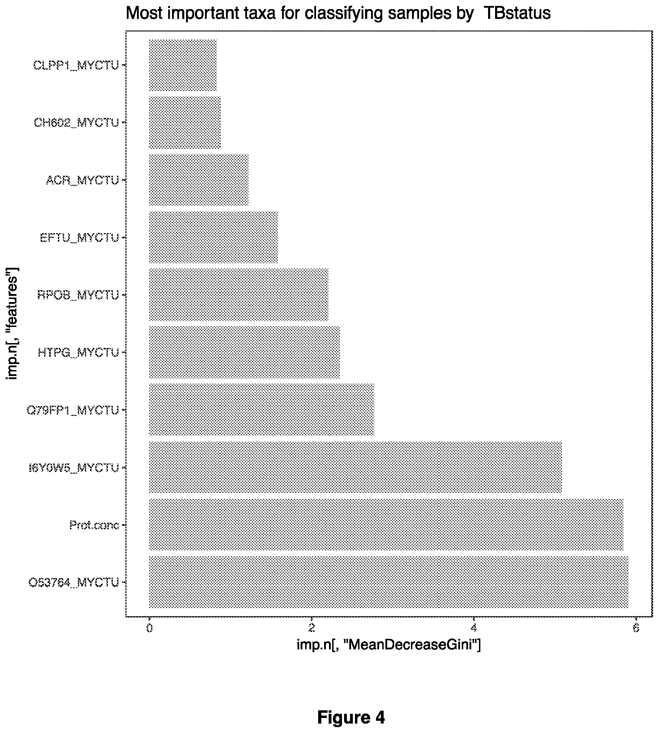

The importance ranking for the top predictors for TB status was determined by the mean decrease in accuracy, with larger decreases on removal of the feature indicating greater predictive power (Figures 2 and 4). The top 7 human biomarkers were SAA1, RBP4, SAA2, A8MUE1, TSP4, CRP and RETN. Random Forest analysis of these seven biomarkers, with 10-fold cross validation, on the training set (41 and 38 TB- and TB+ samples, respectively) resulted in an accuracy of 94% (sensitivity=89%, specificity=99%, positive predictive value (PPV)=97%, negative predictive value (NPV)=91%) (Table 3).

**Table 3: Performance parameters for a protein biosignature consisting of 7 proteins and/or peptides in ruling-in and ruling-out active TB, using a training and test cohort (N = 118).**

| **Performance parameters** | **Training cohort (N** = **79)** | **Test cohort (N = 39)** |
|---|---|---|
| **Accuracy** | 0.94 | 0.90 |
| **Sensitivity (%)** | 89 | 84 |
| **Specificity (%)** | 98 | 95 |
| **PPV (%)** | 97 | 94 |
| **NPV (%)** | 91 | 86 |

Cross-validated error rates associated with a stepwise reduction in biomarkers revealed that four biomarkers would be sufficient to predict TB status with an error rate of ~9%. These top four biomarkers, i.e. SAA1, RPB4, SAA2 and TSP4, were re-tested using a randomly selected validation set (19 and 20 TB+ and TB- samples, respectively), resulting in an accuracy of 92%, PPV of 100%, NPV of 87%, sensitivity of 84% and specificity of 100% (Table 4).

**Table 4: Performance parameters for a protein biosignature comprising SAA1, RPB4, SAA2 and TSP4 in ruling-in and ruling-out active TB (N = 39).**

| | |
|---|---|
| **Sensitivity (%)** | **84** |
| **Specificity (%)** | 100 |
| **PPV (%)** | 100 |
| **NPV (%)** | 87 |

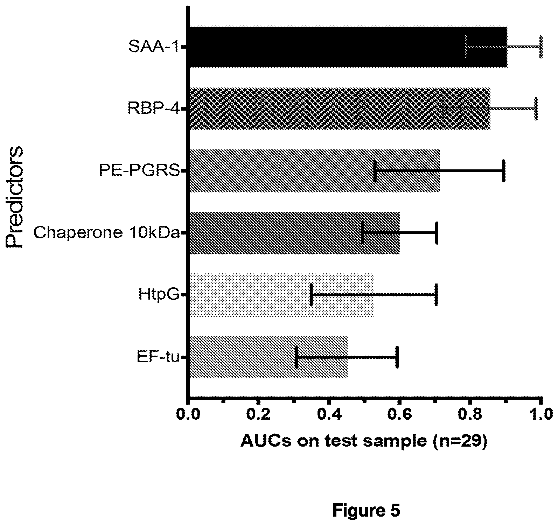

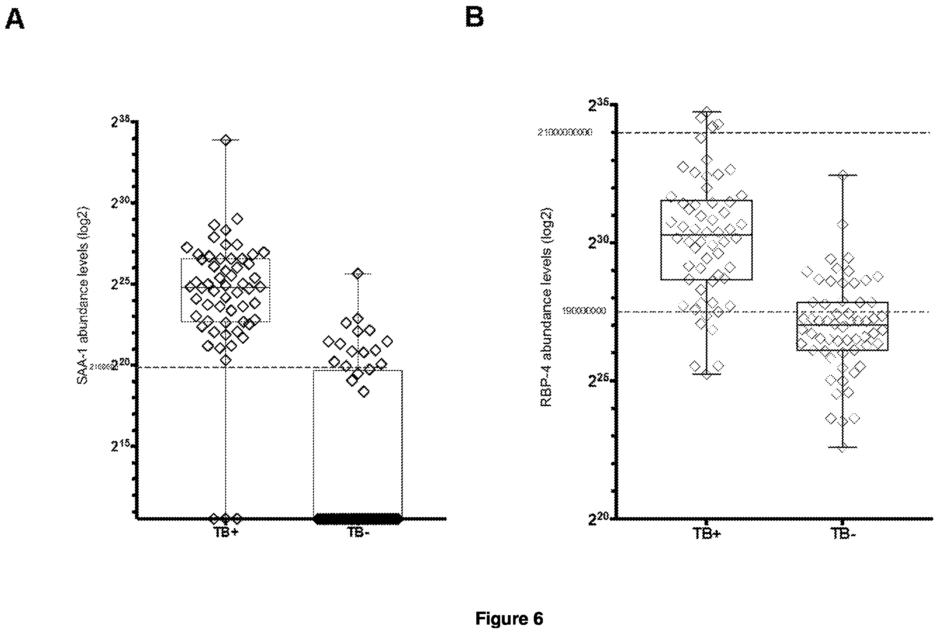

Combinatorial and multidimensional biosignatures comprising two human host-specific biomarkers (SAA1 and RBP4), and SAA1, RBP4 and two to four *M*. *tuberculosis* biomarkers were also evaluated for ruling-in and ruling-out TB, and these were compared to a biosignature of SAA1 only (Figures 5 and 6). The biosignatures were:
SAA1, RBP4, HtpG, PE_PGRS28, 10kDa chaperonin, EFTU_MYCTU;
SAA1, RBP4, HtpG, PE_PGRS28; and
SAA1, RBP4.

The Random Forest analysis results for these biosignatures are shown in Table 5.

**Table 5: The performance of a human and Mycobacterium tuberculosis-derived combinatorial and multidimensional biosignature comprising up to 6 proteins and/or peptides in ruling-in and ruling-out active TB.**

| **Model** | **AUC(95% Cl)** | **BER** | **F-1** | **Sens.** | **Spec.** | **PPV (95% Cl)** | **NPV (95% Cl)** | **Signature (Model components)** |
|---|---|---|---|---|---|---|---|---|
| | | | | | | | | |
| UBM-2 | 0.990 | 0.107 | 88% | 78.57% | 100% | 1 | 0.83 | 2-human urine proteins: 5AA-1, RBP-4 |
| | (0.972-1.000) | | | | | (0 72-1) | (0.59-0.96) | 4-Mtb urine proteins: Mtp6. 10kDa. PE-PGRS. EF-tu |
| UBM-3 | 0.980 | 0.107 | 88% | 78.57% | 100% | 1 | 0.83 | 2-human urine proteins: SAA-1, RSP-4 |
| | (0.944-1.000) | | | | | (0.72-1) | (0.59-0.96) | 2-Mtb urine proteins: HtpG, PE-PGRS |
| UBM-4 | 0.957 | 0.107 | 78% | 78.57% | 100% | 1 | 0.83 | 2-human urine proteins: SAA-1, RBP-4 |
| | (0.888-1.00) | | | | | (0.72-1) | (0.59-0.96) | |
| UBM-5 | 0.905 | 0.179 | 78% | 64% | 100% | 1 | 0.33 | 1-human protein: SAA-1 |
| | (0.789-1.000) | | | | | (0.72-1) | (0.12-0.62) | |

| | | | | | | | | |
|---|---|---|---|---|---|---|---|---|
| BM, biomarker; UBM, urine biomarker; AUC, area under the curve; BER, balanced error rate; Sens., sensitivity; Spec., specificity; PPV, positive predictive value; NPV, negative predictive value; SAA-1, serum amyloid A; RBP-4, Retinol-binding protein 4, | | | | | | | | |

### Example 2:

### Materials and Methods

### Clinical classification

The above cohort (N=118) was classified into two broader groups according to TB status: TB+ (n=57) and TB- (n=61).

### Data processing

LC-MS data generated as above were analysed using MaxQuant version 1.6.14.0 with match between runs, iBAQ, and LFQ on. The database used for raw data analysis was downloaded from Uniprot in November 2019 and was a combined *Homo sapiens* and *Mycobacterium tuberculosis* H37Rv database, containing 74053 human sequences and 3999 M. *tuberculosis* sequences. The MaxQuant output folder '.txt' was analysed using an in-house QC pipeline in R to assess the quality of the runs. Contaminant and reverse hits were removed from the data.

### Statistical analysis

Statistical testing was carried out on the iBaq values for the individual biomarkers, using Random Forest analysis as well as differential abundance analysis as above, focusing on the following 24 candidate human biomarkers identified in Figure 2:

**Table 6: Top 24 candidate human biomarkers selected for further testing**

| | **Biomarker ID** | **Gene name** |
|---|---|---|
| 1 | SAA1 | SAA1 |
| 2 | Q5VY30 | RBP4 |
| 3 | SAA2 | SAA2 |
| 4 | A8MUE1 | LILRB4 |
| 5 | TSP4 | THBS4 |
| 6 | CRP | CRP |
| 7 | RETN | RETN |
| 8 | LV321 | IGLV3-21 |
| 9 | FABPL | FABP1 |
| 10 | KV117 | IGKV1-17 |
| 11 | E5RGN3 | ATOX1 |
| 12 | AACT | SERPINA3 |
| 13 | E9PNW4 | CD59 glycoprotein |
| 14 | A0A0C4DH68 | IGKV2-24 |
| 15 | I18BP | IL18BP |
| 16 | A0A0A0MT36 | IGKV6D-21 |
| 17 | KVD39 | IGKV1D-39 |
| 18 | AXA81 | ANXA8L1 |
| 19 | HV307 | IGHV3-30 |
| 20 | A0A087X0T8 | CADM1 |
| 21 | CSPG2 | VCAN |
| 22 | ADIPO | ADIPOQ |
| 23 | FHR2 | CFHR2 |
| 24 | A0A0C4DGY8 | ENOPH1 |

### Results

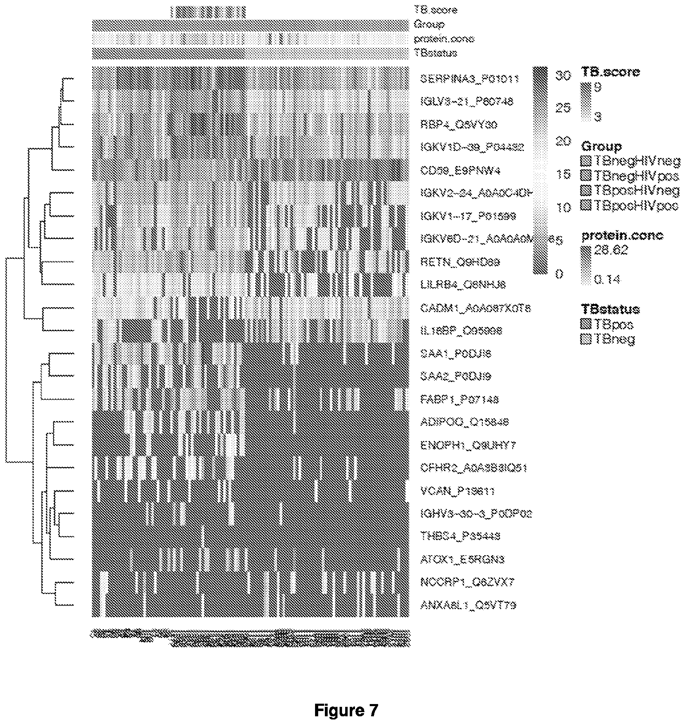

Differential abundance testing was performed on iBAQ values to identify proteins within the set of 24 candidate biomarkers that are significantly differentially abundant between TB+ and TB- individuals (Figure 7).

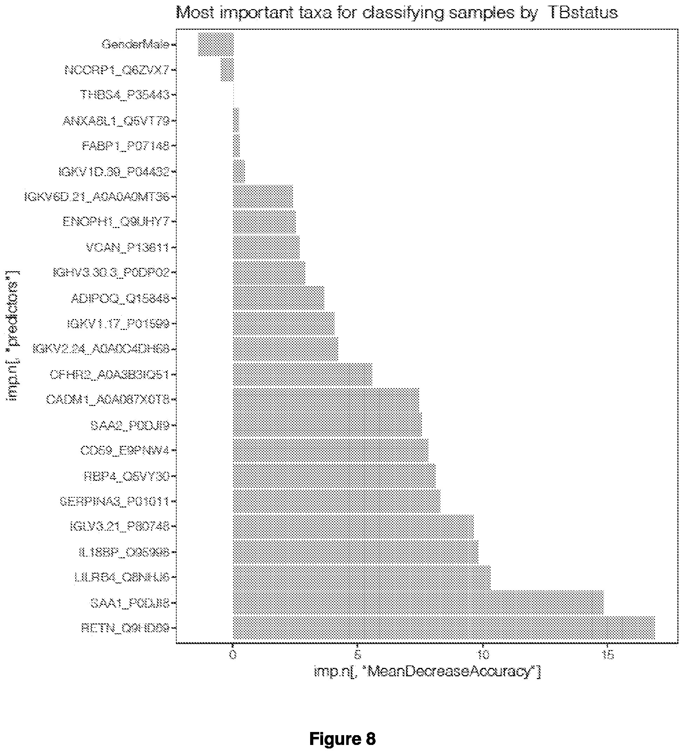

The importance ranking for the top predictors for TB status was determined within the set of 24 candidate biomarkers by the mean decrease in accuracy, with larger decreases on removal of the feature indicating greater predictive power (Figure 8). The top 2 human biomarkers were SAA1 and RETN; the top 3 human biomarkers were SAA1 and RETN combined with either LILRB4 or IL18BP; the top 4 human biomarkers were SAA1, RETN, LILRB4 and IL18BP.

### Analysis of training sets

Random Forests analysis of the 2 biomarker panel, SAA1 and RETN, with 10-fold cross validation, on the training set (38 and 41 TB+ and TB- samples, respectively) resulted in sensitivity=89%, specificity=91%, positive predictive value (PPV)=90%, negative predictive value (NPV)=90%.

Random Forests analysis of the 3 biomarker panel, SAA1, RETN and LILRB4, with 10-fold cross validation, on the training set (38 and 41 TB+ and TB- samples, respectively) resulted in sensitivity=92%, specificity=93%, positive predictive value (PPV)=92%, negative predictive value (NPV)=93%.

Random Forests analysis of the 3 biomarker panel, SAA1, RETN and IL18BP, with 10-fold cross validation, on the training set (38 and 41 TB+ and TB- samples, respectively) resulted in sensitivity=97%, specificity=95%, positive predictive value (PPV)=95%, negative predictive value (NPV)=97%.

Random Forests analysis of the 4 biomarker panel, SAA1, RETN, LILRB4 and IL18BP, with 10-fold cross validation, on the training set (38 and 41 TB+ and TB- samples, respectively) resulted in sensitivity=97%, specificity=95%, positive predictive value (PPV)=95%, negative predictive value (NPV)=97%.

Random Forests analysis of the 7 biomarker panel, SAA1, RETN, LILRB4, IL18BP, SERPINA3, CD59 and CADM1, with 10-fold cross validation, on the training set (38 and 41 TB+ and TB- samples, respectively) resulted in sensitivity=89%, specificity=98%, positive predictive value (PPV)=96%, negative predictive value (NPV)=93%.

Random Forests analysis of the 7 biomarker panel, SAA1, RETN, LILRB4, IL18BP, SERPINA3, CD59 and CFHR2, with 10-fold cross validation, on the training set (38 and 41 TB+ and TB- samples, respectively) resulted in sensitivity=95%, specificity=95%, positive predictive value (PPV)=95%, negative predictive value (NPV)=95%.

Random Forests analysis of the 7 biomarker panel, SAA1, RETN, LILRB4, IL18BP, SERPINA3, IGLV3.21 and RBP4, with 10-fold cross validation, on the training set (38 and 41 TB+ and TB- samples, respectively) resulted in sensitivity=92%, specificity=98%, positive predictive value (PPV)=97%, negative predictive value (NPV)=93%.

### Statistical validation on test sets

These top biomarker panels were then re-tested using a randomly selected validation set (19 and 20 TB+ and TB- samples, respectively), resulting in the accuracy (AUC), sensitivity, specificity, PPV and NPV values shown in Table 7.

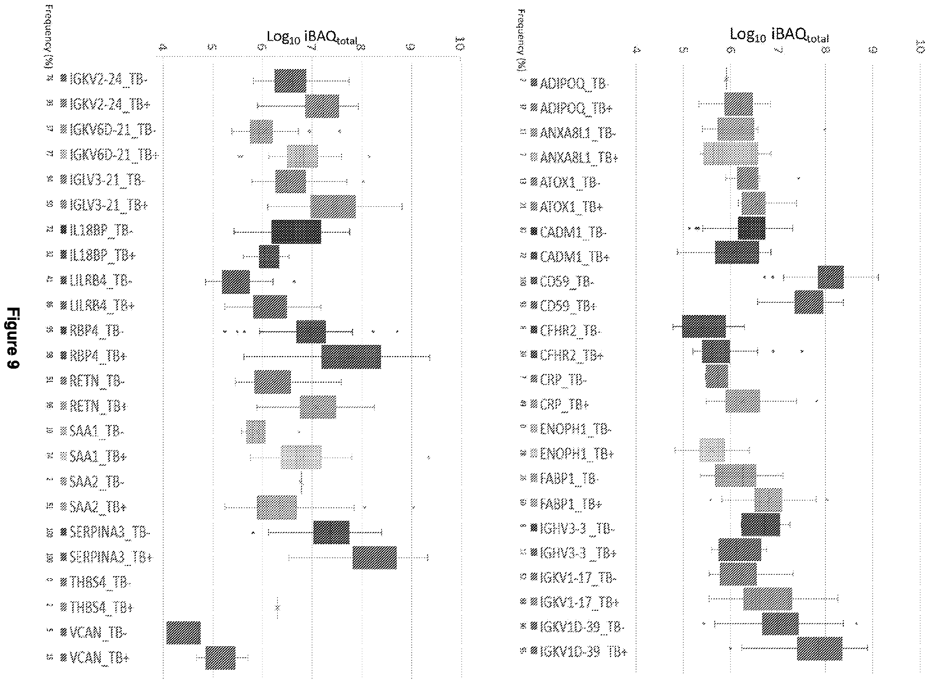

The distribution and frequency of each biomarker from this set was further analysed using box plots (Figure 9), to gain additional insight regarding the utility of the identified biomarker panels.

Ideal biomarkers in a clinically-useful diagnostic panel should be expressed in a high percentage of cases and/or controls and should provide clear differentiation between cases and controls. Based on this data and rationale, combined with the Random Forests analyses (Table 7) and differential abundance testing (Figure 7) described above, the preferred set of human urinary biomarkers for diagnosis of TB disease are drawn from the list comprising SAA1, RETN, LILRB4 (A8MUE1), IL18BP, SERPINA3 (AACT), CD59 (E9PNW4), RBP4 (Q5VY30), IGLV3.21 (LV321) and IGKV1.17 (KV117).

**Table 7: The performance of a human-derived combinatorial and multidimensional biosignature comprising up to 7 proteins in ruling-in and ruling-out active TB.**

| Model | AUC (95% Cl) | Sensitivity | Specificity | PPV | NPV | Signature |
|---|---|---|---|---|---|---|
| UBM6 | 0.85 (0.69-0.96) | 0.82 | 0.87 | 0.86 | 0.84 | 2 human urine proteins: |
| | | | | | | SAA1, RETN |
| UBM7 | 0.89 (0.74-0.97) | 0.92 | 0.85 | 0.86 | 0.91 | 3 human urine proteins: |
| | | | | | | SAA1, RETN, LILRB4 |
| UBM8 | 0.87 (0.73-0.96) | 0.84 | 0.90 | 0.89 | 0.85 | 3 human urine proteins: |
| | | | | | | SAA1, RETN, IL18BP |
| UBM9 | 0.91 (0.77-0.97) | 0.95 | 0.88 | 0.88 | 0.95 | 4 human urine proteins: |
| | | | | | | SAA1, RETN, LILRB4, IL18BP |
| UMB10 | 0.9 (0.76-0.97) | 0.90 | 0.90 | 0.90 | 0.90 | 4 human urine proteins: |
| | | | | | | SAA1, RETN, IL18BP, IGKV1.17 |
| UBM11 | 0.94 (0.80-0.99) | 0.93 | 0.95 | 0.93 | 0.95 | 7 human urine proteins: |
| | | | | | | SAA1, RETN, LILRB4, IL18BP, SERPINA3, CD59, CADM1 |
| UBM12 | 0.95 (0.83-0.99) | 0.89 | 1.00 | 1.00 | 0.91 | 7 human urine proteins: |
| | | | | | | SAA1, RETN, LILRB4, IL18BP, SERPINA3, CD59, CFHR2 |
| UBM13 | 0.97 (0.87-1.0) | 1.00 | 0.95 | 0.95 | 1.00 | 7 human urine proteins: |
| | | | | | | SAA1, RETN, LILRB4, IL18BP, SERPINA3, RBP4, IGLV3.21 |

## Claims

1. A method for diagnosing tuberculosis disease (TB), the method comprising the step of testing a urine sample from a subject for the presence of SAA1 (Serum amyloid A1) and either of RETN (Resistin) or RBP4 (Retinol binding protein).

2. A method according to claim 1, which comprises testing the urine sample for the presence of SAA1 and RETN.

3. A method according to claim 1 or 2, which further comprises testing the urine sample for the presence of one or more additional biomarkers selected from the group consisting of LILRB4 (Leukocyte immunoglobulin-like receptor subfamily B member 4), IL18BP (Interleukin-18-binding protein), SERPINA3 (Serpin family A member 3), CD59 (CD59 glycoprotein), RBP4, IGLV3.21 (Immunoglobulin lambda variable 3.21), IGKV1.17 (Immunoglobulin kappa variable 1-17), O53764_MYCTU (Rv0567) (Probable methyltransferase/ methylase), I6Y0W5_MYCTU (fadE19, Rv2500c) (Acyl-CoA dehydrogenase), Q79FP1_MYCTU (PE_PGRS28, Rv1452c) (PE-PGRS family protein), HTPG_MYCTU (htpG, Rv2299c) (Chaperone protein HtpG), RPOB_MYCTU (rpoB, Rv0667) (DNA-directed RNA polymerase subunit beta), EFTU_MYCTU (tuf, Rv0685) (Elongation factor Tu), ACR_MYCTU (hspX, Rv2031c) (Alpha-crystallin), CH602_MYCTU (groEL2, Rv0440) (60 kDa chaperonin), CLPP1_MYCTU (clpP1, Rv2461c) (ATP-dependent Clp protease proteolytic subunit 1) and 10kDa chaperonin (Rv3418c).

4. A method according to claim 2, which further comprises testing the urine sample for the presence of IL18BP.

5. A method according to either of claims 2 or 4, which further comprises testing the urine sample for the presence of LILRB4.

6. A method according to any one of claims 2, 4 or 5, which further comprises testing the urine sample for the presence of from one to five additional biomarkers selected from the group consisting of SERPINA3, CD59, RBP4, IGLV3.21 and IGKV1.17.

7. A method according to any one of claims 1 to 6, which further comprises testing the urine sample for the presence of from one to four additional biomarkers selected from the group consisting of HtpG, PE_PGRS28, EFTU_MYCTU and 10kDa chaperonin.

8. A method according to any one of claims 3 to 7, which comprises testing the sample for the presence of the following biomarkers:
a) SAA1, RETN and LILRB4;
b) SAA1, RETN and IL18BP;
c) SAA1, RETN, IL18BP and LILRB4;
d) SAA1, RETN, IL18BP and IGKV1.17;
e) SAA1, RETN, IL18BP, LILRB4 and SERPINA3;
f) SAA1, RETN, IL18BP, LILRB4, SERPINA3 and CD59; or
g) SAA1, RETN, LILRB4, IL18BP, SERPINA3 and any two of CD59, RBP4, IGLV3.21, CADM1 and CFHR2.

9. A method according to claim 1, which comprises testing the urine sample for the presence of SAA1 and RBP4.

10. A method according to claim 9, which comprises testing the urine sample for the presence of SAA1, RBP4, HtpG and PE_PGRS28.

11. A method according to claim 10, which comprises testing the urine sample for the presence of SAA1, RBP4, HtpG, PE_PGRS28, EFTU_MYCTU and 10kDa chaperonin.

12. A computer implemented method for diagnosing tuberculosis disease (TB) in a subject, the computer performing steps comprising:
a) receiving inputted subject data comprising values for levels of SAA1 and either or both of RETN and RBP4 in a urine sample from the subject;
b) comparing these values with predetermined values for the biomarkers;
c) determining whether the subject has TB; and
d) displaying information regarding the diagnosis of the subject.

13. A computer implemented method according to claim 12, wherein in step (a), inputted subject data comprising values for levels of one or more other biomarkers in the urine sample is additionally received, wherein the other biomarkers are selected from the group consisting of LILRB4, IL18BP, SERPINA3, CD59, RBP4, IGLV3.21, IGKV1.17, O53764_MYCTU (Rv0567), I6Y0W5_MYCTU (fadE19, Rv2500c), Q79FP1_MYCTU (PE_PGRS28, Rv1452c), HTPG_MYCTU (htpG, Rv2299c), RPOB_MYCTU (rpoB, Rv0667), EFTU_MYCTU (tuf, Rv0685), ACR_MYCTU (hspX, Rv2031c), CH602_MYCTU (groEL2, Rv0440), CLPP1_MYCTU (clpP1, Rv2461c) and CH10_MYCTU (10kDa chaperonin, Rv3418c).

## Patentansprüche

1. Verfahren zur Diagnose einer Tuberkuloseerkrankung (TB), wobei das Verfahren den Schritt des Testens einer Urinprobe eines Probanden auf das Vorhandensein von SAA1 (Serum Amyloid A1) und entweder RETN (Resistin) oder RBP4 (Retinol bindendes Protein) umfasst.

2. Verfahren nach Anspruch 1, das das Testen der Urinprobe auf das Vorhandensein von SAA1 und RETN umfasst.

3. Verfahren nach Anspruch 1 oder 2, das ferner das Testen der Urinprobe auf das Vorhandensein eines oder mehrerer zusätzlicher Biomarker umfasst, die aus der Gruppe ausgewählt sind, die aus LILRB4 (Leukozyten-Immunglobulin-ähnlicher Rezeptor, Unterfamilie B, Mitglied 4), IL18BP (Interleukin-18-bindendes Protein), SERPINA3 (Serpin Familie A, Mitglied 3), CD59 (CD59-Glykoprotein), RBP4, IGLV3.21 (Immunglobulin lambda variabel 3.21), IGKV1.17 (Immunglobulin kappa variabel 1-17), O53764_MYCTU (Rv0567) (Wahrscheinliche Methyltransferase/ Methylase), I6Y0W5_MYCTU (fadE 19, Rv2500c) (Acyl-CoA-Dehydrogenase), Q79FP1_MYCTU (PE_PGRS28, Rv1452c) (PE-PGRS-Familienprotein), HTPG-MYCTU (htpG, Rv2299c) (Chaperonprotein HtpG), RPOB_MYCTU (rpoB, Rv0667) (DNA-gerichtete RNA-Polymerase-Untereinheit beta), EFTU_MYCTU (tuf, Rv0685) (Dehnungsfaktor Tu), ACR_MYCTU (hspX, Rv2031c) (Alpha-Kristallin), CH602-MYCTU (groEL2, Rv0440) (60 kDa Chaperonin), CLPP1_MYCTU (clpP1, Rv2461c) (ATP-abhängige proteolytische Untereinheit 1 der Clp-Protease) und 10kDa Chaperonin (Rv3418c) besteht.

4. Verfahren nach Anspruch 2, das ferner das Testen der Urinprobe auf das Vorhandensein von IL18BP umfasst.

5. Verfahren nach einem der Ansprüche 2 oder 4, das ferner das Testen der Urinprobe auf das Vorhandensein von LILRB4 umfasst.

6. Verfahren nach einem der Ansprüche 2, 4 oder 5, das ferner das Testen der Urinprobe auf das Vorhandensein von einem bis fünf zusätzlichen Biomarkern umfasst, die aus der Gruppe ausgewählt sind, die aus SERPINA3, CD59, RBP4, IGLV3.21 und IGKV1.17 besteht.

7. Verfahren nach einem der Ansprüche 1 bis 6, das ferner das Testen der Urinprobe auf das Vorhandensein von einem bis vier zusätzlichen Biomarkern umfasst, die aus der Gruppe ausgewählt sind, die aus HtpG, PE_PGRS28, EFTU_MYCTU und 10kDa-Chaperonin besteht.

8. Verfahren nach einem der Ansprüche 3 bis 7, das das Testen der Probe auf das Vorhandensein der folgenden Biomarker umfasst:
a) SAA1, RETN und LILRB4;
b) SAA1, RETN und IL18BP;
c) SAA1, RETN, IL18BP und LILRB4;
d) SAA1, RETN, IL18BP und IGKV1.17;
e) SAA1, RETN, IL18BP, LILRB4 und SERPINA3;
f) SAA1, RETN, IL18BP, LILRB4, SERPINA3 und CD59; oder
g) SAA1 , RETN, LILRB4, IL18BP, SERPINA3 und zwei beliebige von CD59, RBP4, IGLV3.21, CADM1 und CFHR2.

9. Verfahren nach Anspruch 1, das das Testen der Urinprobe auf das Vorhandensein von SAA1 und RBP4 umfasst.

10. Verfahren nach Anspruch 9, das das Testen der Urinprobe auf das Vorhandensein von SAA1, RBP4, HtpG und PE_PGRS28 umfasst.

11. Verfahren nach Anspruch 10, das das Testen der Urinprobe auf das Vorhandensein von SAA1, RBP4, HtpG, PE_PGRS28, EFTU_MYCTU und 10kDa-Chaperonin umfasst.

12. Computerimplementiertes Verfahren zur Diagnose einer Tuberkuloseerkrankung (TB) bei einem Probanden, wobei der Computer Schritte ausführt, die umfassen:
a) Empfangen von eingegebenen Probandendaten, die Werte für den Gehalt an SAA1 und entweder RETN oder RBP4 oder beides in einer Urinprobe von dem Probanden umfassen;
b) Vergleichen dieser Werte mit vorgegebenen Werten für die Biomarker;
c) Bestimmen, ob der Proband TB hat; und
d) Anzeigen von Informationen über die Diagnose des Probanden.

13. Computerimplementiertes Verfahren nach Anspruch 12, wobei in Schritt (a) zusätzlich eingegebene Probandendaten empfangen werden, die Werte für die Werte eines oder mehrerer anderer Biomarker in der Urinprobe umfassen, wobei die anderen Biomarker aus der Gruppe ausgewählt sind, die aus LILRB4, IL18BP, SERPINA3, CD59, RBP4, IGLV3.21, IGKV1.17, O53764_MYCTU (Rv0567), I6YOW5_MYCTU (fadE19, Rv2500c), Q79FP1_MYCTU (PEPGRS28, Rv1452c), HTPG_MYCTU (htpG, Rv2299c), RPOB_MYCTU (rpoB, Rv0667), EFTU_MYCTU (tuf, Rv0685), ACR_MYCTU (hspX, Rv2031c), CH602_MYCTU (groEL2, Rv0440), CLPP1_MYCTU (clpP1, Rv2461c) und CH10_MYCTU (10kDa Chaperonin, Rv3418c) besteht.

## Revendications

1. Procédé de diagnostic d'une maladie tuberculeuse (TB), le procédé comprenant l'étape consistant à tester un échantillon d'urine provenant d'un sujet pour détecter la présence de SAA1 (amyloïde sérique A1) et de RETN (résistine) ou de RBP4 (protéine de liaison au rétinol).

2. Procédé selon la revendication 1, qui comprend l'étape consistant à tester l'échantillon d'urine pour détecter la présence de SAA1 et de RETN.

3. Procédé selon la revendication 1 ou 2, qui comprend en outre l'étape consistant à tester l'échantillon d'urine pour détecter la présence d'un ou plusieurs biomarqueurs supplémentaires choisis dans le groupe comprenant LILRB4 (membre 4 de la sous-famille B de récepteur de type immunoglobuline leucocytaire), IL18BP (interleukine-18-protéine de liaison), SERPINA3 (membre 3 de la famille A des Serpines), CD59 (glycoprotéine CD59), RBP4, IGLV3.21 (immunoglobuline lambda variable 3.21), IGKV1.17 (immunoglobuline kappa variable 1-17), O53764_MYCTU (Rv0567) (méthyltransférase probable/méthylase), 16Y0W5_MYCTU (fadE19, Rv2500c) (Acyl-CoA déshydrogénase), Q79FP1_MYCTU (PE_PGRS28, Rv1452c) (protéine de la famille PE-PGRS), HTPG_MYCTU (htpG, Rv2299c) (protéine chaperone HtpG), RPOB_MYCTU (rpoB, Rv0667) (Sous-unité bêta de l'ARN polymérase dirigée par l'ADN), EFTU_MYCTU (tuf, Rv0685) (facteur d'allongement Tu), ACR_MYCTU (hspX, Rv2031c) (Alpha-cristalline), CH602_MYCTU (groEL2, Rv0440) (chaperonine 60 kDa), CLPP1_MYCTU (clpP1, Rv2461c) (sous-unité protéolytique 1 de la protéase Clp dépendante de l'ATP) et chaperonine 10kDa (Rv3418c).

4. Procédé selon la revendication 2, qui comprend en outre l'étape consistant à tester l'échantillon d'urine pour détecter la présence d'IL18BP.

5. Procédé selon l'une quelconque des revendications 2 ou 4, qui comprend en outre l'étape consistant à tester l'échantillon d'urine pour détecter la présence de LILRB4.

6. Procédé selon l'une quelconque des revendications 2, 4 ou 5, qui comprend en outre l'étape consistant à tester l'échantillon d'urine pour détecter la présence de un à cinq biomarqueurs supplémentaires choisis dans le groupe constitué de SERPINA3, CD59, RBP4, IGLV3.21 et IGKV1.17.

7. Procédé selon l'une quelconque des revendications 1 à 6, qui comprend en outre l'étape consistant à tester l'échantillon d'urine pour détecter la présence de un à quatre biomarqueurs supplémentaires choisis dans le groupe constitué de HtpG, PE_PGRS28, EFTU_MYCTU et chaperonine 10 kDa.

8. Procédé selon l'une quelconque des revendications 3 à 7, qui comprend l'étape consistant à tester l'échantillon pour la présence des biomarqueurs suivants :
a) SAA1, REIN et LILRB4 ;
b) SAA1, REIN et IL18BP ;
c) SAA1, REIN, IL18BP et LILRB4 ;
d) SAA1, REIN, IL18BP et IGKV1.17 ;
e) SAA1, REIN, IL18BP, LILRB4 et SERPINA3 ;
f) SAA1, REIN, IL18BP, LILRB4, SERPINA3 et CD59 ; ou
g) SAA1, REIN, LILRB4, IL18BP, SERPINA3 et deux quelconques parmi CD59, RBP4, IGLV3.21, CADM1 et CFHR2.

9. Procédé selon la revendication 1, qui comprend l'étape consistant à tester l'échantillon d'urine pour la présence de SAA1 et de RBP4.

10. Procédé selon la revendication 9, qui comprend l'étape consistant à tester l'échantillon d'urine pour la présence de SAA1, RBP4, HtpG et PE_PGRS28.

11. Procédé selon la revendication 10, qui comprend l'étape consistant à tester l'échantillon d'urine pour la présence de SAA1, RBP4, HtpG, PE_PGRS28, EFTU_MYCTU et chaperonine 10 kDa.

12. Procédé implémenté par ordinateur pour diagnostiquer une maladie tuberculeuse (TB) chez un sujet, l'ordinateur effectuant les étapes consistant à :
a) recevoir des données de sujet entrées comprenant des valeurs pour des niveaux de SAA1 et de l'un ou l'autre ou des deux parmi REIN et RBP4 dans un échantillon d'urine provenant du sujet ;
b) comparer ces valeurs avec des valeurs prédéterminées pour les biomarqueurs ;
c) déterminer si le sujet est atteint de TB ; et
d) afficher des informations concernant le diagnostic du sujet.

13. Procédé mis en œuvre par ordinateur selon la revendication 12, dans lequel à l'étape (a), des données de sujet entrées comprenant des valeurs pour les niveaux d'un ou de plusieurs autres biomarqueurs dans l'échantillon d'urine sont reçues en plus, dans lequel les autres biomarqueurs sont choisis dans le groupe constitué de LILRB4, IL18BP, SERPINA3, CD59, RBP4, IGLV3.21, IGKV1.17, O53764_MYCTU (Rv0567), 16Y0W5_MYCTU (fadE19, Rv2500c), Q79FP1 _MYCTU (PE_PGRS28, Rv1452c), HTPG_MYCTU (htpG, Rv2299c), RPOB_MYCTU (rpoB, Rv0667), EFTU_MYCTU (tuf, Rv0685), ACR_MYCTU (hspX, Rv2031c), CH602_MYCTU (groEL2, Rv0440), CLPP1 _MYCTU (clpP1, Rv2461c) et CH10_MYCTU (chaperonine 10kDa, Rv3418c).
